# EUROPEAN PATENT SPECIFICATION

(11) **EP 0 778 042 B1**
(45) Date of publication and mention of the grant of the patent: **01.03.2000**
(21) Application number: 96308701.0
(22) Date of filing: 02.12.1996
(51) Int. Cl.: A61M 25/09

(54) **Nickel-titanium, lubricious medical catheter wire**
Aus Nickel-Titan bestehender gleitfähiger Katheterdraht
Fil de cathéter lubrifié en nickel-titan

(30) Priority: 04.12.1995 US 566981
(43) Date of publication of application: 11.06.1997
(73) Proprietor: TARGET THERAPEUTICS, INC., Fremont, CA 94538 (US)
(72) Inventor: Kupiecki, David, Cupertino, California 95014 (US)
(74) Representative: Price, Nigel John King

(56) References cited:
- EP-A- 0 141 006
- EP-A- 0 405 823
- EP-A- 0 519 604
- EP-A- 0 550 258
- EP-A- 0 661 073
- WO-A-95/01123
- US-A- 3 841 308
- US-A- 4 991 602
- US-A- 5 304 198
- US-A- 5 417 708

## Description

### FIELD OF THE INVENTION

This invention is a medical device for use in catheterization procedures. More specifically, it is a medical catheter wire for use within a catheter lumen and having a super-elastic metallic core coated with lubricious coating and having a distal region desirably with reduced stiffness that ends in an enlarged distal tip. The device may be used as a valve wire in combination with a single lumen balloon catheter or as a wire pusher in combination with an introducer delivery catheter that coaxially houses a helical vaso-occlusive coil.

### BACKGROUND OF THE INVENTION

Percutaneous catheterization is a procedure that allows for the administration of medical therapy or the taking of diagnostic measurements at a distally remote internal body site without the need for direct surgical access to that target site. The less invasive catheterization procedures allow for medical devices, such as catheters and guidewires, or their assemblies to be inserted into the body at an initial access site that is remote from the target internal site. Such devices or assemblies may then guided through the channels of the body, such as through the vasculature, via X-ray visualization, until the distal end portion of the catheter or guidewire reaches the target site. Treatment or diagnosis is then achieved at the distal end portion of the device via manipulation of the proximal end portion extending outside the patient's body.

One type of medical device useful in many catheterization procedures is the "guidewire." Guidewires are elongate medical devices having metallic cores that are designed to provide a pathway over which catheters are guided through the bends, loops, and branches forming the blood vessels within the body. One method of using a guidewire to direct the catheter through the tortuous paths of these lumenal systems involves the use of a torqueable guidewire which is directed as a unit with the catheter from a body access point such as the femoral artery to the tissue region containing the target site. The guidewire typically has a small bend its distal end, and may be guided by alternately rotating and advancing the guidewire along the small vessel pathway to the desired target. The guidewire and the catheter are advanced by alternately moving the guidewire along a distance in the vessel pathway, holding the guidewire in place, and then advancing the catheter coaxially along the axis of the guidewire until it reaches the portion of the guidewire already advanced farther into the human body.

The difficulty in accessing remote body regions, e.g., the body's periphery or the soft tissues within the body such as the brain and the liver, present apparent challenges to guidewire designs. A catheter and its coaxially housed guidewire must be both flexible to allow the combination to follow the complicated path through the tissue, and yet stiff enough to allow the distal end of the catheter to be manipulated by the physician from the external access site. It is common that the catheter is as long as a meter or more. It is also common that the guidewire is longer than the catheter such that the guidewire extends both proximally and distally of the coaxial catheter to allow for guidewire manipulation and sub-selective tracking in the vasculature.

To meet the performance requirements as just described, guidewires may be constructed having tapers or transitions of materials along the guidewire length to vary the wire flexibilities. Some guidewires have included super-elastic alloy cores, hydrophilic coatings, and enlarged distal tips in order to achieve the requisite guidewire functionalities.

Examples of some catheter guidewires used in guiding a catheter through the human vasculature that have variable flexibility constructions may be found in the following references: U.S. Patent Nos. 3,789,841; 4,545,390; 4,619,274; and. 5,095,915.

Other guidewire disclosures have included use of various super-elastic alloys in an attempt to achieve some of the noted sub-selecting and guiding functions. Examples of specific Ni-Ti alloys are disclosed in U.S. Patent Nos. 3,174,851; 3,351,463; and 3,753,700. Examples of some references disclosing guidewires using super-elastic alloys are found in the following references: U.S. Patent 4,925,445; WO91/15152 (to Sahatjian et al. and owned by Boston Scientific Corp.); U.S. Patent 4,665,906; U.S. Patent 4,969,890; U.S. Patent 4,984,581; U.S. Patent No. 5,069,226; U.S. Patent No. 5,171,383; and Published European Patent Application 0,515,201-A1.

Other references disclosing the use of hydrophilic polymers, with or without a polymeric tie layer, to increase the surface lubricity of guidewires having superelastic metal alloy core construction include: U.S. Patent No. 5,443,907 to Slaikeu, et. al; U.S. Patent No. 5,129,890 to Bates, et. al.; Published European Patent Application 0,519,604-A2. A further example of a guidewire having a specifically beneficial shape memory alloy for its metallic core, a hydrophilic coating, and optionally a polymeric tie layer between the two is disclosed in pending U.S. Patent Application Serial No. 08/346,143 to Palermo, et. al.

References disclosing guidewires having an enlarged wire tip on a superelastic alloy core that is hydrophilically coated include U.S. Patent No. 5,243,996 to Hall. Hall discloses a wire guide having a mandrel of superelastic metallic material, such as nitinol, and having a reduced-diameter portion in its distal region. A flexible radiopaque platinum coil is attached at the distal region of the mandrel and coaxially surrounds a portion of the distal region. At least one polymer layer may be used to coat the mandrel for increased lubricity. The lubricious covering may be a hydrophilic coating on the coil and a majority of the mandrel, leaving the proximal portion uncoated for ease of physician manipulation.

A further example of a guidewire having a nickel-titanium alloy core and an enlarged tip, although not disclosing use of a hydrophilic polymer, is found in U.S. Patent 4,991,602 to Amplatz et al. Amplatz et. al. suggests a flexible guidewire made up of a shape memory alloy, such as the nickel-titanium alloy known as nitinol. The guidewire is one having a single diameter throughout its midcourse, is tapered toward each end, and has a bead or ball at each of those ends. The bead or ball is selected to allow ease of movement through the catheter into the vasculature. The guidewire is symmetrical so that a physician cannot make a wrong choice in determining which end of the guidewire to insert into the catheter. The patent suggests that wound wire coils at the guidewire tip are undesirable. The patent further suggests the use of a polymeric coating (PTFE) and an anticoagulant.

A variety of wire devices, other than guidewires, have also been designed for optimally performing functions in medical device assemblies other than providing a rail for guiding over-the-wire catheters to remote internal body spaces. For example, a control wire or deflector wire for use in a guideable catheter assembly is disclosed in U.S. Patent No. 5,419,340 to Stevens. Stevens discloses the control wire as having a stainless steel core wire, a coating that may be a hydromer, and a ball tip that may be approximately .032" and rounded to promote axial travel within a catheter without binding. The control wire is resiliently biased in a linear orientation for the purposes of selectively deflecting a curved distal portion of a catheter into relaxed and unrelaxed positions.

Another type of useful medical wire device, not necessarily a guidewire, has been disclosed to provide a valving mechanism when assembled with certain types of "single-lumen" balloon catheters. "Single lumen" balloon catheters generally have one lumen that facilitates balloon inflation and at the same time is co-axial with the guidewire. A valve mechanism is usually provided on the wire (or on the catheter) such that a fluid seal may be selectively achieved between the wire and the catheter. The wire is slidable within the lumen and may be advanced and torqued relatively independently of the catheter in order to select and track to remote sites. Yet, the lumen may be tightly sealed onto the wire via the valve mechanism provided for balloon inflation when desired.

Examples of balloon catheters that generally have one inflation lumen that is also coaxially disposed about a guide wire having a valve member thereon are disclosed in U.S. Patent Nos. 4,813,934 to Engelson, et al.; 5,437,632 to Engelson, et al.; 5,304,198 to Samson; and 5,304,198 to Walker, et al.

A further type of medical wire device is a wire pusher used for advancing a device through the delivery lumen of a delivery catheter and out a distal port thereof and into an internal body space. More specifically, wire pushers have been used to advance a type of artificial vaso-occlusion device, pushable vaso-occlusion coils, through a delivery catheter lumen and out a distal port to occlude an internal body space. The significance of this type of wire pusher device to the current invention will be discussed in more detail immediately below.

Artificial vaso-occlusion is a procedure wherein an implantable medical device is delivered, usually through the delivery lumen of a delivery catheter, into a remote internal body space to occlude that space. Examples of medical malformations that have been occluded using artificial vaso-occlusion techniques are aneurysms, arterio-venous malformations, and vessels leading to tumorous tissues.

A highly respected implantable device for artificial vaso-occlusion is the vaso-occlusive coil. Various types of vaso-occlusion coil devices and related methods are known, primarily including detachable coils and pushable coils. Pushable coils use devices known as pushers to advance the coil through a delivery catheter and into the body space for occlusion. Pushable vaso-occlusive devices are most relevant to the current invention.

The detachable vaso-occlusion coil is generally detachably integrated with or attached at its proximal end with an elongate pusher and is delivered to a desired location for occlusion, by means of the pusher, through a delivery catheter which terminates at or near the entrance zone of the desired location. Once the coil is extended out of the delivery catheter and into the desired location, the coil is detached from the pusher and left as an implant. The detachment means may be mechanical, such as the type described in U.S. Patent No. 5,250,071 to Palermo, or may be electrolytic, such as the type described in U.S. Patent No. 5,122,136 to Guglielmi et al.

"Pushable" vaso-occlusion devices or coils are not integrated with a pusher but are independent devices. Such devices are generally pre-packaged in pre-loaded introducer catheters as cartridges and discharged therefrom into a distal delivery catheter. In one type of pushable vaso-occlusion coil, pressurized fluid may hydraulically deliver the pre-loaded implant, such as is disclosed in the following references: U.S. Patent No. 5,133,731 to Butler et al; U.S. Patent No. 5,167,624 to Butler et al; and pending U.S. Patent Application Serial No. 08/413,970 for "Liquid Coils With Secondary Shape", filed March 30, 1995.

One pushable vaso-occlusion coil assembly using a non-integrated pusher is disclosed in U.S. Patent No. 5,382,260 for "Embolization Device and Apparatus Including an Introducer Cartridge and Method for Delivering the Same," to Dormandy, et al. Dormandy '260 discloses an introducer cartridge having a distal extremity with a tapered tip and a hub mounted on a proximal extremity. The cartridge is introduced into a coil delivery catheter in a catheter system as described earlier, and a stylet is used to push the embolization device out of the introducer cartridge into a passage in the coil delivery catheter. A wire that Dormandy characterizes as a guide wire is then used to push the coil through the coil delivery catheter until it advances beyond the catheter tip and into the desired site for occlusion. Such a wire, however, does not "guide" other devices coaxially over its shaft or otherwise function as a "guidewire" either in the classical sense or as that term has been herein defined in this specification.

Another pushable vaso-occlusion coil that is pre-loaded in an introducer cartridge and is delivered via a pusher device is disclosed in U.S. Patent No. 5,382,259 to Phelps et al. Phelps discloses a vaso-occlusion coil that may be continuous or segmented, onto which a fibrous, woven or braided, tubular covering or element is attached. Phelps further discloses that the coil devices may be supplied prepackaged in a sterile cannula which is adapted to engage the proximal end of a delivery catheter. To deliver the Phelps et al. coil device, the distal end of a delivery catheter is placed adjacent to a desired occlusion site, and the coil-containing cannula is placed into engagement with the proximal end of the catheter. The coils are then transferred from the cannula lumen into the catheter lumen by exerting a force on the proximal end of the coil. The reference discloses use of a flexible type of pusher device to push the coil through the delivery catheter and out its distal end to the desired site.

Another vaso-occlusion coil that is pre-loaded in an introducer cartridge and is delivered via a separate pusher is disclosed in U.S. Patent No. 4,994,069 to Ritchart et al. Ritchart et al. discloses a coiled wire for use in small-vessel vaso-occlusion and having a convoluted space filling conformation when in a relaxed condition, a linear configuration when in a stretched condition, and a memory from the stretched to the relaxed condition when released from a delivery catheter into a vessel. Ritchart et al. provides the vaso-occlusion wire in a prepackaged form in a sterile cannula which is adapted to engage the proximal end of the delivery catheter. The cannula is attached to the delivery catheter and the wire is transferred into the delivery catheter by a short wire. A pusher is disclosed for advancing the wire through the delivery catheter.

Another example of an embolic coil that is pre-loaded in an introduction device as a cartridge is described in Dormandy '260. The patent discloses a co-axial telescoping arrangement including a femoral artery sheath, a guiding catheter, and a coil delivery catheter. A C-shaped embolization device such as a coil is provided in an introducer cartridge made of clear transparent plastic such as a radiation sterilizable polycarbonate. A tubular member of the cartridge is bendable but relatively rigid so that it can be utilized as an introducer. A distal extremity of the cartridge is provided with a tapered tip and a hub is mounted on the proximal extremity, the hub also being formed of clear radiation sterilizable polycarbonate.

A pre-loaded tube for delivery of a vein-branch blocking member is also disclosed in U.S. Patent No. 5,342,394 to Matsuno et al. Matsuno discloses an apparatus and method for blocking a vein branch comprising a vein-branch blocking member pushed out of a delivery tube with a push-out member and into the vein. The outer tube is bent and slidably receives an inner tube with a distal storing portion filled with a vein-branch blocking member. A push-out member pushes out the blocking member in the vein branch. The pre-loaded inner tube is flexible and has a proximal end that is fixed with a grip that is adapted to engage the proximal end of the outer tube in various embodiments. The distal shape of the outer tube is between 60 and 120 degrees and allows the inner tube to be easily inserted in the vein branch branching from the lateral wall of the vein. An embodiment discloses an articulation means for the distal bend, but no way to have a straight end if desired. The proximal end of the tube, however, is straight but is not adapted to be used distally nor is the distal shaped end adapted to straighten and be interchangeably used proximally.

None of the cited references disclose a medical catheter wire having a superelastic metallic core that is partially coated with a hydrophilic coating and that has a distal wire section that is substantially straight, unshapeable, and having a distal reduction in wire stiffness and a larger wire tip profile than the distal wire section.

Nor do these references disclose such a medical catheter wire assembled as a valve wire in combination with a single-lumen type of balloon catheter, wherein the enlarged wire tip forms a fluid seal with a valve seat of the balloon catheter.

Nor do these references disclose such a medical catheter wire assembled as a wire pusher in combination with an introducer delivery catheter that co-axially houses a target delivery device, such as a vaso-occlusive coil, the enlarged wire tip being adapted to cooperate with a proximal device end during advancement of the target delivery device through the introducer delivery catheter.

EP-A-0 519 604 (mentioned above) is the document upon which the preamble of claim 1 is based.

According to the present invention there is provided a medical catheter wire assembly for use within a medical catheter lumen, comprising:
a superelastic metallic core having a proximal wire portion, an intermediate wire portion, a substantially straight and unshapeable distal wire portion being more flexible than said intermediate wire portion, and a lubricious coating at least partially covering said intermediate and distal wire portions to form a distal wire section and an intermediate wire section ;
wherein
said lubricious coating forms an outer diameter on said intermediate wire section which, at the interface between said lubricious coating and said proximal wire portion, is substantially equal to and continuous with the outer diameter of said metallic core of said proximal wire portion at said interface; and
said assembly further comprises an enlarged wire tip adjacent to said distal wire section and having a larger wire tip profile than said distal wire section and being substantially unshapeable.

In one embodiment the medical catheter wire assembly further comprises:
a balloon catheter having a proximal coupler, an expandable balloon, a balloon tip distal of said balloon and with a distal wire port, a wire lumen extending between said proximal coupler and said distal wire port and fluidly coupling said expandable balloon with said proximal coupler, and a valve seat located interiorly of said wire lumen and having a reduced inner diameter from said wire lumen,
said reduced inner diameter of said valve seat being adapted to removably engage said enlarged wire tip in an interference fit to form a fluid seal such that fluid interior of said wire lumen may be pressurized to cause balloon expansion.

In another embodiment the medical catheter wire assembly further comprises:
a delivery catheter having a proximal delivery coupler, a distal delivery port, and a polymeric delivery shaft defining a delivery lumen extending between said proximal delivery coupler and said distal delivery port, said delivery lumen having an inner diameter adapted to slideable receive said enlarged wire tip and intermediate and distal wire sections; and
a target device being slideable within said delivery lumen and having a proximal device end portion adapted to cooperate with said enlarged wire tip in sliding said target device through the delivery lumen and out the distal delivery port.

### BRIEF DESCRIPTION OF THE DRAWINGS

FIG. 1 shows a cut-away side view of the medical catheter wire of the current invention.

FIG. 2 is a partial side view of a further medical catheter wire embodiment wherein a polymeric tie layer is shown between the superelastic metal alloy core and a lubricious hydrophilic coating.

FIG. 3 shows a cut-away side view of a balloon catheter/valve wire assembly embodiment of the current invention, wherein the medical catheter wire of FIG. 1 is shown assembled as a valve wire in combination with one type of single-lumen balloon catheter.

FIG. 4 shows a perspective view of a medical device delivery system embodiment of the current invention, wherein the medical catheter wire of FIG. 1 and a vaso-occlusive coil are shown in phantom within the delivery lumen of a delivery catheter.

### DESCRIPTION OF THE INVENTION

### Medical Catheter Wire

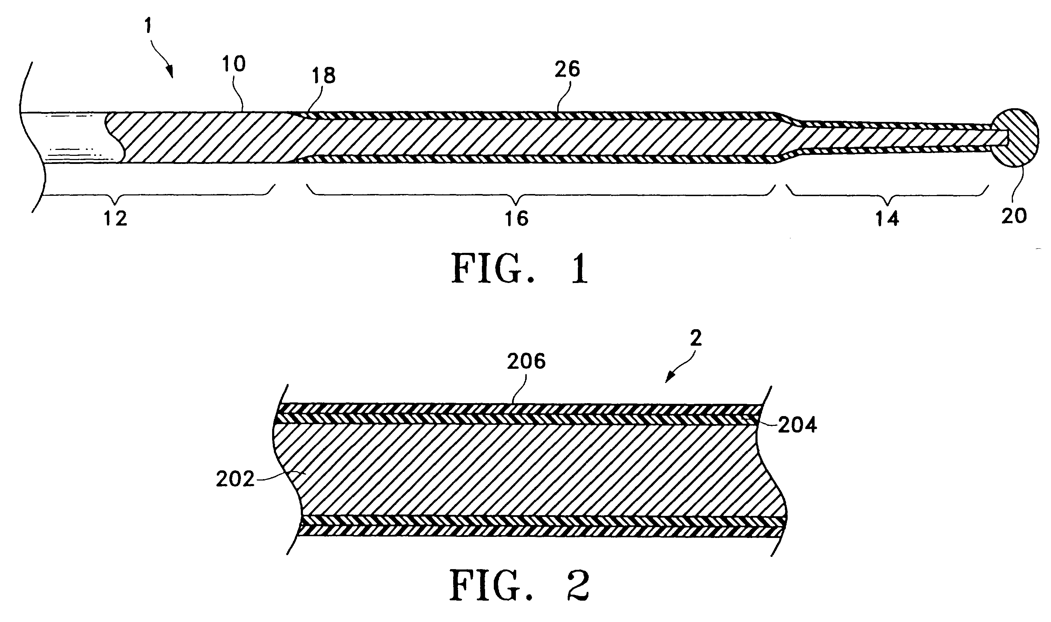

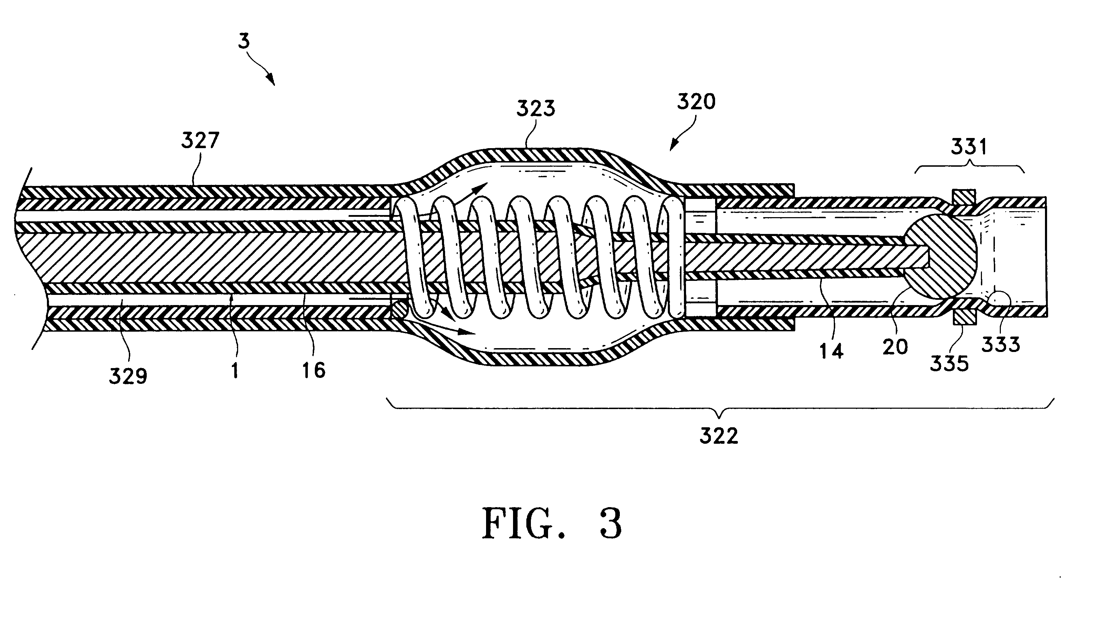

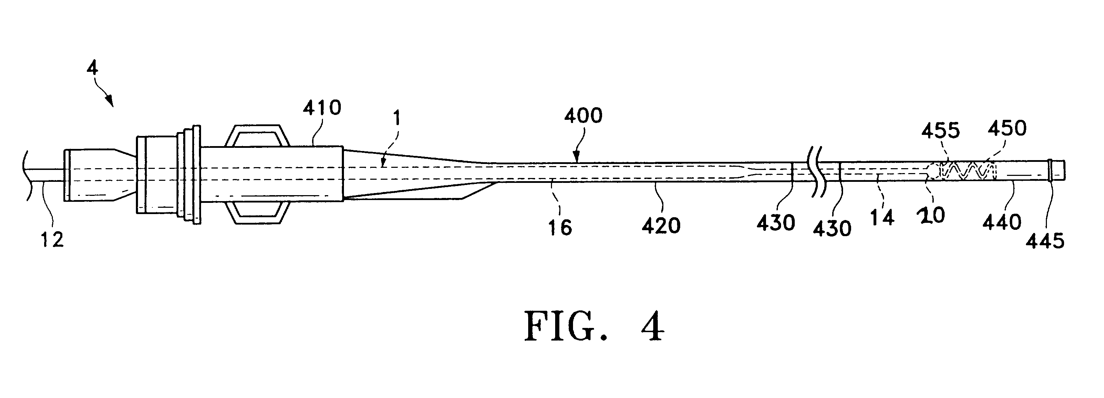

FIG. 1 shows a cut-away side view of a medical catheter wire embodiment of the current invention, wherein medical catheter wire (1) is shown to have a proximal wire section (12), an intermediate wire section (16), a distal wire section (14), and an enlarged wire tip (20). Medical catheter wire (1) is also made up of a superelastic metallic core (10) formed of the alloys described below and has a total length typically between about 50 and 300 centimeters. The features of medical catheter wire (1), as shown in FIG. 1 and described in detail below, allow use either as a valve wire in a balloon catheter/valve wire assembly (as shown in FIG. 3) or as a wire pusher in a medical device delivery system (as shown in FIG. 4).

The proximal wire section (12) preferably has a uniform diameter (along its length) of about 0.0254 to 0.0889 cm (0.010 to 0.035 inches), preferably 0.0254 to 0.04064 cm (0.010 to 0.016 inches). The distal wire section may be relatively more flexible than the proximal section. If so, the distal wire section (14) extends for 3 to 30 centimeters or more of the distal end of the medical catheter wire (1). Any reduction in flexibility may be achieved in a variety of ways, e.g., through changes in material along the wire length (such as in the particular superelastic alloy composition of the metallic core in the section) or through changes in geometry (such as through a ground reduction in the superelastic core outer diameter in the section). In any case, the distal wire section (14), although flexible, is substantially straight and unshapeable, particularly desirable aspects of the wire as a valve wire and a wire pusher in the preferred embodiments of FIGS. 3 and 4.

Also shown in FIG. 1 is intermediate wire section (16). The intermediate wire section comprises a portion of the metallic core having a slightly reduced outer diameter. The intermediate wire portion has a coating on its outer surface of such thickness as to form a continuous outer diameter with proximal wire section (12). However, the intermediate wire section (16) may be continuously tapered, may have a number of tapered sections or sections of differing diameters, or may be of a uniform diameter along its length. If intermediate wire section (16) is of a generally uniform diameter, the medical catheter wire core may neck down as is seen at (18).

Distal wire section (14) of the medical catheter wire (1) is also shown ending distally in an unshapeable, enlarged wire tip (20), which is shown to have a larger profile than distal wire section (14). The unshapeability of enlarged wire tip (20), similar to that of the distal wire section (14), allows for the medical catheter wire to be optimally durable under the compressive push forces of use as a valve wire or as a wire pusher. The enlarged wire tip profile also optimizes use of the device as a valve wire or wire pusher, since the geometry of the distal wire end must be adapted to engage a cooperating valve seat or proximal device end, as is respectively required in the embodiment of FIG. 3. The particular benefits of these features will be explained in more detail below. The enlarged wire tip (20) may also be radiopaque and made from materials including but not limited to platinum, tungsten, gold, or alloys thereof. Such radiopaque metals allow knowledge of the position of enlarged wire tip (20) during the process of inserting the medical catheter wire through a coaxial catheter disposed in the vasculature.

The enlarged wire tip (20) may be formed on medical catheter wire (1) using a variety of methods that result in an unshapeable tip. One acceptable method involves soldering a radiopaque metal element onto the very tip of the metal core and then melting the metal element, such as with a laser or using electrical discharge techniques as may be apparent to one of ordinary skill. Such radiopaque metal element may be a metal coil placed coaxially over the end of the core and then attached as just described. Or, the metal element may be a metallic band coaxially placed and secured in a similar manner. The result of these methods produces an enlarged wire tip (20) that is preferably substantially solid, which optimizes radiopacity and unshapeability of the tip.

The coating on all or part of the intermediate wire section (16) and distal wire section (14) is preferably a thin layer of polymeric material (26) to improve these sections lubricity without adversely affecting the flexibility of the medical catheter wire. As is discussed below, when using hydrophilic polymers as the coating material, it is often desirable to use a polymeric tie layer on the medical catheter wire core.

The medical catheter wire of the present invention, such as the medical catheter wire (1) embodiment of FIG. 1 mentioned above, may be used as a valve wire in a single-lumen type of balloon catheter, or as a wire pusher in a delivery catheter which is made up of an elongate polymeric tubular member having ports at proximal and distal ends. Such catheters are (again) about 50 to 300 centimeters in length, typically between about 100 and 200 centimeters in length. Generally, the polymeric tubular shafts of such catheters have a relatively stiff proximal section which extends along a major portion of the catheter length and one or more relatively flexible distal sections which provide greater ability of the catheter to track a guidewire through sharp bends and turns encountered as the catheter is advanced through the tortuous paths found in the vasculature. Suitable catheters in the preferred catheter/wire assembly embodiments of the present invention are discussed in more detail in FIGS. 3 and 4.

The unshapeable design provided for distal end section (14) and enlarged wire tip (20) allows for the medical catheter wire (1) to be an optimal "push wire" for use internally with such catheters. The term "unshapeability" as defined herein as the ability of such a medical catheter wire to confront another device's surface within the body and push against it, as a part of a particular method, without plastic deformation to the wire. This is a preferred quality either in pushing against a proximal side of a single-lumen valve seat to form a fluid seal for balloon inflation (as is shown and described for assembly 3 of FIG. 3) or in pushing against a target device for delivery within a lumen of a delivery catheter (as is shown and described for assembly 4 of FIG. 4). Similarly for such "push wire" functionality, it is preferred that the distal wire section (14) and enlarged wire tip (20) be substantially straight, allowing for such push forces as just described to be most efficiently transmitted to the confronting end of enlarged wire tip (20) with minimized prolapse. The medical catheter wire (1) thus has no steering or subselecting function that are required of guidewires; otherwise the wire would be shaped or shapeable and may not provide the beneficial pushing qualities just described.

Guidewires made of certain alloys, particularly Ni-Ti alloys, have super-elastic properties and retain those properties during traversal through the catheter when disposed in the vasculature. Yet such guidewires are sufficiently pliable that they provide the physician using the medical catheter wire with enhanced "feel" or feedback. The preferred alloys do not incur significant unrecovered strain during use, such as when bent within catheters in tortuous anatomy or when under compressive forces of pushing against a valve seat or against a target delivery device within a delivery catheter lumen.

The material used in the medical catheter wires of this invention are of shape memory alloys which exhibit super-elastic/pseudo-elastic shape recovery characteristics. Various acceptable alloys showing these characteristics are known. For instance, alloys disclosed in U.S. Patent Nos. 3,174,851; 3,351,463; and 3,753,700 would be acceptable. The alloy disclosed in the '700 patent may be particularly desirable among those disclosures because of the increased pushability concomitant with the higher modulus of the material disclosed therein as having an increased iron content. These metal alloys are characterized by their ability to be transformed from an austenitic crystal structure to a stress-induced martensitic (SIM) structure at certain temperatures, and return elastically to the austenitic structure when the stress is removed. These alternating crystalline structures provide the alloy with its super-elastic properties. One such well-known alloy, nitinol, is a nickel-titanium alloy. It is readily commercially available and undergoes the austenite-SIM-austenite transformation at a variety of temperature ranges between -200C and 300C.

These alloys are especially suitable because of their capacity to elastically recover almost completely to the initial configuration once the stress is removed. Typically there is little plastic deformation, even at relatively high strains. This allows the medical catheter wire to undertake substantial bends as it passes through the delivery catheter in the body's vasculature, and yet return to its original shape once a bend has been traversed without retaining any hint of a kink or a bend.
Nevertheless, compared to similar stainless steel medical catheter wires, less force need be exerted against the interior wall of the delivery lumen to deform the medical catheter wire of the invention along the desired path through the delivery catheter, thereby reducing friction against the coaxial catheter.

To achieve the desired result of high strength and enhanced durability even while allowing feedback to the attending physician during use, the physical parameters of the preferred alloys and metal working methods disclosed in U.S. Patent No. 5,409,015 to Palermo, et. al. are highly desirable in the present invention.

The coating provided on the metallic core wire in the intermediate and distal wire sections may comprise one or more layers of a polymeric material. The coating is applied typically to enhance the lubricity of the medical catheter wire core during its traversal of the delivery catheter lumen. The coating may be applied by dipping or spraying or by similar process with such materials as polysulfones, polyfluorocarbons (such as TEFLON), polyolefins such as polyethylene, polypropylene, polyesters (including polyamides such as the NYLON's), and polyurethanes; their blends and copolymers such as polyether block amides (e.g., PEBAX).

One preferred coating for the core wire in the intermediate wire section (16) and distal wire section (14) is shown in cross section in FIG. 2. FIG. 2 shows a typical guide wire core section (2) having a superelastic metallic core (202), a polymeric tie layer (204), and a lubricious coating (206), which is most preferably a hydrophilic coating. The material composition and coating methods for forming this coated wire assembly may also be similar to the various embodiments disclosed in pending U.S. Patent Serial No. 08/346,143 to Palermo, et al. Preferred compositions include polymeric materials containing polyvinylpyrrolidone or polyethyleneoxide.

### Balloon Catheter/Valve Wire Assembly

FIG. 3 shows the medical catheter wire (1) of FIG. 1 assembled as a valve wire in combination with one type of "single-lumen" balloon catheter (320), forming balloon/wire assembly (3) .

In this embodiment, the single-lumen balloon catheter design shown may be similar to that disclosed in U.S. Patent No. 5,304,198 to Samson. Inflation shaft (327) of balloon catheter (320) is shown to provide an wire lumen (329) adapted to slideably receive medical catheter wire (1) and also that fluidly couples a proximal inflation coupler (not shown) with expandable balloon (323) (which is shown to be in an expanded state). A portion of medical catheter wire (1) is shown to be coaxially housed within wire lumen (329), and is shown extending coaxially under balloon (323), and distally through balloon catheter tip region (331).

In the balloon catheter tip region (331) is a valve seat (333), which is shown in FIG. 3 to be formed underneath a radiopaque tip marker (335). This valve seat (333) is designed to have a tight tolerance with the profile of enlarged wire tip (20), forming an interference fit therewith. This interference fit forms a barrier to distal fluid flow from the interior space of balloon (323), such that the balloon may be controllably inflated at elevated pressures. Preferably, such valve seat is formed of a compliant polymer and of lower inner diameter that the wire tip profile such that the enlarged wire tip (20) may be pushed coaxially into the valve seat in a press-fit to form a mechanically compressive seal to fluid flow.

In addition, the proximal inflation coupler (not shown) of balloon catheter (320) preferably comprises an adjustable seal around medical catheter wire (1) at intermediate wire section (16) or the proximal wire section (not shown) to provide a barrier to proximal fluid flow during balloon inflation. For instance, a conventional type of rotating hemostatic valve mechanism may be provided at the proximal inflation coupler.

The medical catheter wire (1) in FIG. 3 is shown to have enlarged wire tip (20) abutting a proximal edge of valve seat (333). This arrangement would be achieved, for instance, where balloon catheter (320) is advanced distally into an internal body site over a conventional guidewire, with the guidewire subsequently removed and replaced by the novel valve wire for balloon inflation. The design of medical catheter wire (1) purely as a valve wire in this assembly may be optimized for forming the seal with valve seat (333), without the need for steerability, and atraumatic trackability design requirements found in standard guidewires. For example, the more narrow functionality may allow for a much stiffer distal wire section (14) proximally of enlarged wire tip (20) than a guidewire would allow. Such increased stiffness may allow for more push force at the enlarged wire tip (20) such that a tighter interference fit may be achieved at the valve seat (333). This tighter fit, in turn, may allow for higher inflation pressures to be achieved before the valve seal is compromised. Additionally, the super-elastic alloy core, together with the preferred hydrophilic coating, are designed to optimize the ability to advance the wire through the single-lumen and push against the valve seat (333) without permanently deforming the wire.

FIG. 3 also shows distal wire section (14) having a reduced outer diameter relative to the intermediate wire section (16), the transition to the reduced outer diameter being shown beneath expandable balloon (323). However, it may be preferable to lengthen the reduced outer diameter distal wire section (16) so that it traverses the balloon and extends even proximally within the inflation shaft (327) region when the enlarged wire tip (20) forms the seal with valve seat (333). In such a configuration, a larger intralumenal clearance is created within the inflation lumen (which is also the wire lumen) which may increase inflation/deflation times of the balloon. Additionally, such lengthened region of profile reduction may allow for a tapered outer diameter for the distal portions of inflation shaft (327) and the other more distal balloon catheter components shown.

### Medical Device Delivery Assembly

FIG. 4 is a perspective view of medical catheter wire (1) of FIG. 1 in a medical device delivery assembly of the current invention. In this embodiment, the novel medical catheter wire of the current invention is assembled as a wire pusher to delivery a medical device through the delivery lumen of a delivery catheter. More specifically to the particular embodiment of FIG. 4, medical catheter wire (1) is shown in phantom to be slideably within the coaxial delivery lumen of delivery catheter (400), wherein enlarged wire tip (20) is shown to proximally abut a proximal coil end (455) of a vaso-occlusive coil (450), which is in this case the target device for delivery. Delivery catheter (400) of assembly (4) may be an "over-the-wire" type of delivery catheter that generally is guided to a desired internal body site by riding on its delivery lumen coaxially over a guidewire. One type of a highly beneficial "over-the-wire" delivery catheter that would be suitable in the embodiment of assembly (4) of the current invention is guidewire is described in U.S. Patent No. 4,739,768 to Engelson. A further type of delivery catheter that may be a suitable component to assembly (4) a flow-directable type of delivery catheter, such as that described in U.S. Patent No. 5,336,205 to Zenzen. These delivery catheters generally have a stepwise reduction in stiffness, such that there is a flexible distal region (440) for tracking in tortuous anatomy, and a relatively more stiff proximal region (420) for physician manipulation and transmission of proximal push forces to distal catheter regions. One or more intermediate sections may also be provided, such as is shown at (430) in FIG. 4. Additionally, a proximal delivery coupler (410) and a distal tip marker (445) are preferred components of delivery catheter 400, as may be apparent to one of ordinary skill.
Vaso-occlusive coil (450) preferably is a non-detachable, pushable type of vaso-occlusion coil, such as that disclosed in U.S. Patent Nos.5,382,259; 5,382,260; 4,994,069; and 5,342,394. Another desirable type of non-detachable vaso-occlusion coil that may be useful in the present invention is also shown in pending U.S. Patent Application Design Serial No. 29/037,001.

In general, such pushable vaso-occlusion coils are helically wound wires that have a primary helical geometry that forms a primary helix lumen with a primary helix inner diameter and a primary helix outer diameter. The ends of such helical coils are usually filled or otherwise terminated with a soldered, welded, or otherwise rounded metallic ball or cap. The vaso-occlusion coil is often provided with a secondary geometry, which may also be a secondary helix, having a secondary inner diameter and a secondary outer diameter.

Examples of known commercial vaso-occlusive coil embodiments that may be used in this invention have primary helix outer diameters between about 0.02032 and 0.0889 cm (0.008" and 0.035"), wherein the delivery catheters adapted for delivering such coils into the body are closely toleranced above those dimensions. Enlarged wire tip (20) should be adapted of dimension and shape to confront the rounded end of vaso-occlusion coil (450). Preferably, enlarged wire tip (20) has a rounded end to aid in smooth slideable delivery within the delivery lumen of the delivery catheter. Also preferred, the wire tip profile of enlarged wire tip (20) is equal to or less than the primary helix outer diameters, such as being in the range of 0.01524 and 0.0254 cm (.006" - .010") for use with a 0.0254 cm (.010") primary helix outer diameter, or being in the range of 0.03556 - 0.04572 cm (.014"-.018") for use with a 0.04572 cm (.018") primary helix outer diameter.

In preferred mode of operation for the assembly (4) of FIG. 4, vaso-occlusion coil (450) is provided pre-loaded in a sterile kit within an introducer cannula. The delivery catheter of the assembly is percutaneously introduced into the body and advanced translumenally into a remote internal body space. A distal end of the cannula is coaxially introduced into the proximal hub of the delivery catheter and a separate plunger may be used to eject the pre-loaded coil from the cannula and into the delivery catheter lumen. Or, alternatively, the novel wire pusher of the present invention may be used for this purpose.

Once the coil is introduced into the delivery catheter lumen, the wire pusher is advanced until the enlarged wire tip confronts the proximal device end of the coil. Continued advancement of the wire pusher advances the coil along the delivery lumen until it is expelled from a distal port of the delivery catheter and into the desired internal body space for artificial occlusion.

The above is a detailed description of particular embodiments for the invention. Any combination of the disclosed embodiments is contemplated as within the scope of the present invention. It is also recognized that departures from the disclosed embodiments may be made and obvious modifications will occur to a person skilled in the art without departing from scope of the invention.

## Claims

1. A medical catheter wire assembly for use within a medical catheter lumen, comprising:
a superelastic metallic core (10,202) having a proximal wire portion, an intermediate wire portion, a substantially straight and unshapeable distal wire portion being more flexible than said intermediate wire portion, and a lubricious coating (26, 206) at least partially covering said intermediate and distal wire portions to form a distal wire section (14) and an intermediate wire section (16) ;
characterised in that:
said lubricious coating (26, 206) forms an outer diameter on said intermediate wire section (16) which, at the interface between said lubricious coating and said proximal wire portion, is substantially equal to and continuous with the outer diameter of said metallic core (10, 102) of said proximal wire portion at said interface ; and
said assembly further comprises an enlarged wire tip (20) adjacent to said distal wire section (14) and having a larger wire tip profile than said distal wire section and being substantially unshapeable.

2. The medical catheter wire assembly of claim 1, wherein said superelastic metallic core (10, 102) is an alloy of nickel and titanium.

3. The medical catheter wire assembly of claim 2, wherein said alloy of nickel and titanium is nitinol.

4. The medical catheter wire assembly of any one of the preceding claims, wherein said distal wire section (14) has a smaller outer diameter than said intermediate wire section (16).

5. The medical catheter wire assembly of any one of the preceding claims, wherein said lubricious coating (26, 206) is a hydrophilic coating.

6. The medical catheter wire assembly of any one of the preceding claims, further comprising a polymeric tie layer (204) between said superelastic metallic core (202) and said lubricious coating (206).

7. The medical catheter wire assembly of any one of the preceding claims, wherein said enlarged wire tip (20) is comprised of a radiopaque material.

8. The medical catheter wire assembly of claim 7, wherein said radiopaque material is a metal.

9. The medical catheter wire assembly of claim 8, wherein said metal is chosen from the group consisting of platinum, tungsten, and gold.

10. The medical catheter wire assembly of any one of the preceding claims, wherein said enlarged wire tip (20) is substantially spherical.

11. The medical catheter wire assembly of any one of the preceding claims, further comprising:
a balloon catheter (320) having a proximal coupler, an expandable balloon (323), a balloon tip distal of said balloon and with a distal wire port, a wire lumen (329) extending between said proximal coupler and said distal wire port and fluidly coupling said expandable balloon with said proximal coupler, and a valve seat (333) located interiorly of said wire lumen and having a reduced inner diameter from said wire lumen,
said reduced inner diameter of said valve seat (333) being adapted to removably engage said enlarged wire tip (20) in an interference fit to form a fluid seal such that fluid interior of said wire lumen may be pressurized to cause balloon expansion.

12. The medical catheter wire assembly of claim 11, wherein said valve seat (333) is comprised of a compliant polymeric material and has a valve seat inner diameter less than said enlarged wire tip profile, said enlarged wire tip (20) being adapted to coaxially enter said valve seat in a press fit to mechanically form said fluid seal.

13. The medical catheter wire assembly of any one of claims 1 to 10, further comprising:
a delivery catheter (400) having a proximal delivery coupler, a distal delivery port, and a polymeric delivery shaft defining a delivery lumen extending between said proximal delivery coupler and said distal delivery port, said delivery lumen having an inner diameter adapted to slideable receive said enlarged wire tip (20) and intermediate and distal wire sections (14, 16); and
a target device (450) being slideable within said delivery lumen and having a proximal device end portion (455) adapted to cooperate with said enlarged wire tip (20) in sliding said target device through the delivery lumen and out the distal delivery port.

14. The medical catheter wire assembly of claim 13, wherein said target device (450) is a vaso-occlusive implant adapted for artificially occluding a body space.

15. The medical catheter wire assembly of claim 14, wherein said vaso-occlusive implant (450) is a vaso-occlusive coil comprised of a helically wound wire, being slideable within said delivery lumen, and having a helix inner diameter smaller than said wire tip profile.

16. The medical catheter wire assembly of claim 15, wherein said vaso-occlusive coil (450) has an expanded coil diameter when in a radially relaxed state that is greater than said delivery lumen inner diameter, said vaso-occlusive coil being radially compressible from said radially relaxed state to have a compressed coil diameter and being so compressed when disposed within said delivery lumen such that a force is exerted against the delivery lumen wall defining said delivery lumen.

## Patentansprüche

1. Medizinische Katheterdrahtanordnung zur Verwendung im Lumen eines medizinischen Katheters, umfassend:
einen superelastischen Metallkern (10, 202) mit einem proximalen Drahtabschnitt, einem mittleren Drahtabschnitt, einem im wesentlichen geraden und unverformbaren distalen Drahtabschnitt, der biegsamer ist als der mittlere Drahtabschnitt, und einem gleitfähigen Überzug (26, 206), der den mittleren und den distalen Drahtabschnitt wenigstens teilweise bedeckt, um einen distalen Drahtabschnitt (14) und einen mittleren Drahtabschnitt (16) zu bilden;
dadurch gekennzeichnet, daß:
der gleitfähige Überzug (26, 206) auf dem mittleren Drahtabschnitt (16) einen Außendurchmesser bildet, der an der Grenze zwischen dem gleitfähigen Überzug und dem proximalen Drahtabschnitt im wesentlichen gleich dem Außendurchmesser des Metallkerns (10, 202) des proximalen Drahtabschnitts an der Grenze ist und diesen fortsetzt; und
die Anordnung des weiteren eine vergrößerte Drahtspitze (20) umfaßt, die sich im Bereich des distalen Drahtabschnitts (14) befindet und ein größeres Drahtspitzenprofil besitzt als der distale Drahtabschnitt und im wesentlichen unverformbar ist.

2. Medizinische Katheterdrahtanordnung nach Anspruch 1, bei der der superelastische Metallkern (10, 202) eine Legierung aus Nickel und Titan ist.

3. Medizinische Katheterdrahtanordnung nach Anspruch 2, bei der die Legierung aus Nickel und Titan Nitinol ist.

4. Medizinische Katheterdrahtanordnung nach einem der vorhergehenden Ansprüche, bei der der distale Drahtabschnitt (14) einen kleineren Außendurchmesser besitzt als der mittlere Drahtabschnitt (16).

5. Medizinische Katheterdrahtanordnung nach einem der vorhergehenden Ansprüche, bei der der gleitfähige Überzug (26, 206) ein hydrophiler Überzug ist.

6. Medizinische Katheterdrahtanordnung nach einem der vorhergehenden Ansprüche, des weiteren umfassend eine polymere Verbindungsschicht (204) zwischen dem superelastischen Metallkern (202) und dem gleitfähigen Überzug (206).

7. Medizinische Katheterdrahtanordnung nach einem der vorhergehenden Ansprüche, bei der die vergrößerte Drahtspitze (20) aus einem strahlenundurchlässigen Material besteht.

8. Medizinische Katheterdrahtanordnung nach Anspruch 7, bei der das strahlenundurchlässige Material ein Metall ist.

9. Medizinische Katheterdrahtanordnung nach Anspruch 8, bei der das Metall ausgewählt ist aus der Gruppe umfassend Platin, Wolfram und Gold.

10. Medizinische Katheterdrahtanordnung nach einem der vorhergehenden Ansprüche, bei der die vergrößerte Drahtspitze (20) im wesentlichen kugelförmig ist.

11. Medizinische Katheterdrahtanordnung nach einem der vorhergehenden Ansprüche, des weiteren umfassend:
einen Ballonkatheter (320) mit einem proximalen Kopplungsstück, einem aufweitbaren Ballon (323), einer distal von dem Ballon angeordneten Ballonspitze mit einer distalen Drahtöffnung, einem Drahtlumen (329), das sich zwischen dem proximalen Kopplungsstück und der distalen Drahtöffnung erstreckt und eine Fluidverbindung herstellt zwischen dem aufweitbaren Ballon und dem proximalen Kopplungsstück, und einem Ventilsitz (333), der sich innerhalb des Drahtlumens befindet und gegenüber dem Drahtlumen einen verminderten Innendurchmesser besitzt,
wobei der verminderte Innendurchmesser des Ventilsitzes (333) dazu geeignet ist, in einem Preßsitz lösbar in die vergrößerte Drahtspitze (20) einzugreifen, um eine Fluiddichtung herzustellen, so daß das Fluid im Inneren des Drahtlumens mit Druck beaufschlagt werden kann, um die Aufweitung des Ballons zu bewirken.

12. Medizinische Katheterdrahtanordnung nach Anspruch 11, bei der der Ventilsitz (333) aus einem nachgiebigen polymeren Material besteht und einen Innendurchmesser besitzt, der kleiner ist als der Umriß der vergrößerten Drahtspitze, wobei die vergrößerte Drahtspitze (20) in einem Preßsitz koaxial in den Ventilsitz eindringen kann, um auf mechanischem Wege die Fluiddichtung herzustellen.

13. Medizinische Katheterdrahtanordnung nach einem der Ansprüche 1 bis 10, des weiteren umfassend:
einen Verabreichungskatheter (400) mit einem proximalen Verabreichungskopplungsstück, einer distalen Verabreichungsöffnung und einem polymeren Verabreichungsschaft, der ein Verabreichungslumen begrenzt, welches sich zwischen dem proximalen Verabreichungskopplungsstück und der distalen Verabreichungsöffnung erstreckt, wobei das Verabreichungslumen einen Innendurchmesser besitzt, der die vergrößerte Drahtspitze (20) und den mittleren und den distalen Drahtabschnitt (14, 16) verschieblich aufnehmen kann; und
eine Zielvorrichtung (450), die in dem Verabreichungslumen verschieblich ist und einen proximalen Vorrichtungsendabschnitt (455) besitzt, der mit der vergrößerten Drahtspitze (20) zusammenwirken kann, indem er die Zielvorrichtung durch das Verabreichungslumen und aus der distalen Verabreichungsöffnung hinaus schiebt.

14. Medizinische Katheterdrahtanordnung nach Anspruch 13, bei der die zielvorrichtung (450) ein gefäßverschließendes Implantat ist, das einen Körperhohlraum künstlich verschließen kann.

15. Medizinische Katheterdrahtanordnung nach Anspruch 14, bei der das gefäßverschließende Implantat (450) eine gefäßverschließende Spirale ist, die aus einem in dem Verabreichungslumen verschieblichen, spiralförmig gewickelten Draht besteht und einen Innendurchmesser der Helix besitzt, der kleiner ist als der Umriß der Drahtspitze.

16. Medizinische Katheterdrahtanordnung nach Anspruch 15, bei der die gefäßverschließende Spirale (450) im radial entspannten Zustand einen vergrößerten Spiralendurchmesser besitzt, der größer ist als der Innendurchmesser des Verabreichungslumens, wobei die gefäßverschließende Spirale ausgehend von dem radial entspannten Zustand radial komprimierbar ist, so daß sie einen komprimierten Spiralendurchmesser besitzt und bei Anordnung in dem Verabreichungslumen so komprimiert ist, daß eine Kraft auf die das Verabreichungslumen begrenzende verabreichungslumenwand ausgeübt wird.

## Revendications

1. Ensemble de fil de cathéter médical destiné à être utilisé à l'intérieur d'un conduit de cathéter médical, comprenant :
un coeur métallique superélastique (10, 202) comportant une partie de fil proximale, une partie de fil intermédiaire, une partie de fil distale informable et sensiblement droite, qui est plus souple que ladite partie de fil intermédiaire, et un revêtement lubrifiant (26, 206) recouvrant au moins partiellement lesdites parties de fil intermédiaire et distale de façon à former une section de fil distale (14) et une section de fil intermédiaire (16) ;
caractérisé en ce que :
ledit revêtement lubrifiant (26, 206) forme un diamètre extérieur sur ladite section de fil intermédiaire (16), qui, au niveau de l'interface entre ledit revêtement lubrifiant et ladite partie de fil proximale, est sensiblement égal au diamètre extérieur dudit coeur métallique (10, 102) de ladite partie proximale au niveau de ladite interface et continu avec celui-ci ; et
ledit ensemble comprend de plus une pointe de fil agrandie (20) adjacente à ladite section de fil distale (14) et ayant un profil de pointe de fil plus grande que celui de ladite section de fil distale, et étant sensiblement informable.

2. Ensemble de fil de cathéter médical selon la revendication 1, dans lequel ledit coeur métallique superélastique (10, 102) est un alliage de nickel et de titane.

3. Ensemble de fil de cathéter médical selon la revendication 2, dans lequel ledit alliage de nickel et de titane est du nitinol.

4. Ensemble de fil de cathéter médical selon l'une quelconque des revendications précédentes, dans lequel ladite section de fil distale (14) a un diamètre extérieur inférieur à celui de ladite section de fil intermédiaire (16).

5. Ensemble de fil de cathéter médical selon l'une quelconque des revendications précédentes, dans lequel ledit revêtement lubrifiant (26, 206) est un revêtement hydrophile.

6. Ensemble de fil de cathéter médical selon l'une quelconque des revendications précédentes, comprenant de plus une couche de liaison polymère (204) entre ledit coeur métallique superélastique (202) et ledit revêtement lubrifiant (206).

7. Ensemble de fil de cathéter médical selon l'une quelconque des revendications précédentes, dans lequel ladite pointe de fil agrandie (20) se compose d'un matériau radio-opaque.

8. Ensemble de fil de cathéter médical selon la revendication 7, dans lequel ledit matériau radio-opaque est un métal.

9. Ensemble de fil de cathéter médical selon la revendication 8, dans lequel ledit métal est choisi parmi le groupe comprenant le platine, le tungstène et l'or.

10. Ensemble de fil de cathéter médical selon l'une quelconque des revendications précédentes, dans lequel ladite pointe de fil agrandie (20) est sensiblement sphérique.

11. Ensemble de fil de cathéter médical selon l'une quelconque des revendications précédentes, comprenant de plus :
un cathéter à ballonnet (320) comportant un coupleur proximal, un ballonnet dilatable (323), une pointe de ballonnet distale par rapport audit ballonnet et avec un orifice de fil distal, un conduit de fil (329) s'étendant entre ledit coupleur proximal et ledit orifice de fil distal et couplant vis-à-vis des fluides ledit ballonnet dilatable audit coupleur proximal, et un siège de soupape (333) disposé à l'intérieur dudit conduit de fil et ayant un diamètre intérieur réduit par rapport à celui dudit conduit de fil,
ledit diamètre intérieur réduit dudit siège de vanne (333) étant adapté pour venir en prise de façon amovible avec ladite pointe de fil agrandie (20) en un joint à ajustement serré de façon à former un joint étanche vis-à-vis des fluides de telle sorte que le fluide à l'intérieur dudit conduit de fil puisse être mis sous pression de façon à provoquer la dilatation du ballonnet.

12. Ensemble de fil de cathéter médical selon la revendication 11, dans lequel ledit siège de soupape (333) se compose d'un matériau polymère élastique et a un diamètre intérieur de siège de soupape inférieur audit profil de pointe de fil agrandie, ladite pointe de fil agrandie (20) étant adaptée pour entrer coaxialement dans ledit siège de soupape en un ajustage serré pour former mécaniquement ledit joint d'étanchéité vis-à-vis des fluides.

13. Ensemble de fil de cathéter médical selon l'une quelconque des revendications 1 à 10, comprenant de plus :
un cathéter de délivrance (400) comportant un coupleur de délivrance proximal, un orifice de délivrance distal, et un arbre de délivrance polymère définissant un conduit de délivrance s'étendant entre ledit coupleur de délivrance proximal et ledit orifice de délivrance distal, ledit conduit de délivrance ayant un diamètre intérieur adapté pour recevoir de façon coulissante ladite pointe de fil agrandie (20) et lesdites sections de fil intermédiaire et distale (14, 16) ; et
un dispositif cible (450) pouvant coulisser à l'intérieur dudit conduit de délivrance et comportant une partie d'extrémité de dispositif proximale (455) adaptée pour coopérer avec ladite pointe de fil agrandie (20) en faisant coulisser ledit dispositif cible à travers le conduit de délivrance et hors de l'orifice de délivrance distal.

14. Ensemble de fil de cathéter médical selon la revendication 13, dans lequel ledit dispositif cible (450) est un implant vaso-occlusif adapté pour occlure artificiellement un espace corporel.

15. Ensemble de fil de cathéter médical selon la revendication 14, dans lequel ledit implant vaso-occlusif (450) est un serpentin vaso-occlusif composé d'un fil enroulé hélicoïdalement, pouvant coulisser à l'intérieur dudit conduit de délivrance, et ayant un diamètre intérieur de spirale inférieur à celui dudit profil de pointe de fil.

16. Ensemble de fil de cathéter médical selon la revendication 15, dans lequel ledit serpentin vaso-occlusif (450) a un diamètre de serpentin dilaté lorsqu'il est dans un état radialement relâché qui est supérieur audit diamètre intérieur de conduit de délivrance, ledit serpentin vaso-occlusif étant radialement compressible à partir dudit état radialement relâché de façon à avoir un diamètre de serpentin comprimé, et étant comprimé de telle sorte, lorsqu'il est disposé à l'intérieur dudit conduit de délivrance, qu'une force soit exercée contre la paroi de conduit de délivrance définissant ledit conduit de délivrance.
